# EUROPEAN PATENT SPECIFICATION

(11) **EP 1 849 468 B1**
(45) Date of publication and mention of the grant of the patent: **05.12.2012**
(21) Application number: 07112823.5
(22) Date of filing: 25.02.2003
(51) Int. Cl.: A61P 11/06, A61P 11/08, A61K 9/72, A61K 31/44, A61K 31/495

(54) **Pharmaceutical composition comprising roflumilast and levocetirizine**
Pharmazeutische Zusammensetzung enthaltend Roflumilast und Levocetirizin
Composition pharmaceutique comprenant de roflumilast et de levocetirizine

(30) Priority: 06.03.2002 EP 02004987
(43) Date of publication of application: 31.10.2007
(62) Divisional of application: 03708130.4
(73) Proprietor: Nycomed GmbH, 78467 Konstanz (DE)
(72) Inventor: WEIMAR, Christian, 78467, Konstanz (DE); BUNDSCHUH, Daniela, 8272, Ermatingen (CH); BEUME, Rolf, 78465, Konstanz (DE); WOLLIN, Stefan-Lutz, 88339 Bad Waldsee (DE)
(74) Representative: Wild, Robert

(56) References cited:
- EP-A- 1 005 865
- WO-A-01/30777
- WO-A-03/000289
- US-A- 5 962 464
- ANDRI L ET AL: "Combined treatment of allergic rhinitis with terfenadine and nimesulide, a non-steroidal antiinflammatory drug" ALLERGIE ET IMMUNOLOGIE, NOUVELLES EDITIONS MEDICALES FRANCAISES, PARIS, FR, vol. 24, no. 8, October 1992 (1992-10), pages 313-314,317-319, XP002102388 ISSN: 0397-9148
- HATZELMANN A ET AL: "ANTI-INFLAMMATORY AND IMMUNOMODULATORY POTENTIAL OF THE NOVEL PDE4 INHIBITOR ROFLUMILAST IN VITRO" JOURNAL OF PHARMACOLOGY AND EXPERIMENTAL THERAPEUTICS, AMERICAN SOCIETY FOR PHARMACOLOGY AND, US, vol. 297, no. 1, 2001, pages 267-279, XP001024814 ISSN: 0022-3565
- WANG D Y ET AL: "Effect of cetirizine, levocetirizine, and dextrocetirizine on histamine-induced nasal response in healthy adult volunteers." ALLERGY APR 2001, vol. 56, no. 4, April 2001 (2001-04), pages 339-343, XP002450371 ISSN: 0105-4538

## Description

### Field of application of the invention

The present invention relates to combinations of pharmaceutically active substances for use in the treatment of respiratory diseases.

The substances used in the combinations according to the invention are known active compounds from the PDE4 and PDE3/4 inhibitors class and active compounds from the class of the histamine receptor antagonists. More specifically, the present invention relates the combined use of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and [2-[4-[(R)-p-chloro-alpha-phenylbenzyl)-1-piperazinyl]ethoxy]-acetic acid [INN: LEVOCETIRIZINE] or a pharmaceutically acceptable derivative thereof in the treatment of respiratory diseases.

### Known technical background

In the international application WO03/000289 compositions are described which comprise a PDE4 inhibitor and a H1-receptor antagonist and the use of these compositions for the manufacture of a medicament for the treatment of respiratory diseases. In the European patent application EP1005865 pharmaceutical compositions comprising Nimesulide and Cetirizine are disclosed. In Allergie et Immunologie, Vol 24, No 8, 1992, pp 313-319, Andri L. et al have described a combination of Nimesulide with Terfenadine for the treatment of allergic rhinitis. In J. Pharmacol. Exp. Therapeut. Vol 297, No 1, 2001, pp 267-279, Hatzelmann A. et al. have described the anti-inflammatory and immunomodulatory potential of the novel PDE4 inhibitor Roflumilast in vitro. In Allergy, April 2001, vol. 56, no. 4, pages 339-343, Wang D.Y. et al. have described the effect of cetirizine, levocetirizine, and dextrocetirizine on histamine-induced nasal response in healthy adult volunteers.

### Description of the invention

Asthma is a common inflammatory disease of the respiratory tract, accounting for 1 - 3% of all office visits, 500,000 hospital admissions per year and more pediatric hospital admissions than any other single illness in the US. Annually, more than 5000 children and adults die of asthma attacks in the United States (William E. S.; Goodmann Gilmann A.:The pharmacological Basis of Therapeutics, 9th Edition, pp. 152 & 659-682, Mc Graw Hill, New York 1996).

Asthma can no longer be viewed simply as a reversible airway obstruction. It should instead be considered primarily as an inflammatory illness that has bronchial hyperactivity and bronchospasm as its results. Allergen specific immunoglobulin E (IgE) is bound to the mast cells via Fc receptors. It is a fragment obtained by papain digestion of immunoglobulin molecules and contains most of the antigenic determinants. When an allergen comes into contact with IgE, the mast cells are activated and release a number of inflammatory mediators, which include granule contents like histamine, proteases, heparin, and tumor necrosis factor (TNF), a variety of lipid membrane derived molecules like prostaglandins, leukotrienes and platelet activating factor (PAF), and a number of cytokines like intedeukin (IL)-1, 3, 4, 5, 6 and 8 and chemokines. An enormous variety of mediators are released which have more than one potent effect on airway inflammation.

As a result of vasodilation, increased vasopermeability and increased endothelial adhesiveness towards leukocytes further leads to an influx of inflammatory cells like lymphocytes, eosinophils and macrophages from blood circulation into the tissues. This in turn leads to the release of mediators which have further inflammatory effects (Rao A. R. et al. Recent Perspectives in the design of antiasthmatic agents, Pharmazie, 55, 7, 475-482, 2000).

Thus, it can be understood that it is unlikely that drugs affecting a single mediator can satisfactorily treat the disease alone. As asthma is one of the major diseases affecting mankind, there is a need to develop drugs which can affect a wide variety of mediators.

Therefore, it is the object of the present invention to make available respiratory tract therapeutics which fulfill the following conditions:
- Favorable simultaneous influence on several of the inflammatory mediators
- Marked bronchorelaxation and -dilatation
- Good oral availability
- Minor side effects
- Good suitability for long-term therapy
- Favorable influence on bronchial hyperreactivity

It has now been found that the combined use of the PDE4 or a PDE3/4 inhibitor 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and the histamine receptor antagonist [2-[4-[(R)-p-chloro-alpha-phenylbenzynyl)-1-piperazinyl]ethoxy]-acetic acid [INN: LEVOCETIRIZINE] or a pharmaceutically acceptable derivative thereof outstandingly fulfills the above-mentioned conditions.

The invention thus relates to the combined use of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and [2-[4-[(R)-p-chloro-alpha-phenylbenzyl)-1-piperazinyl]ethoxy]-acetic acid [INN: LEVOCETIRIZINE] or a pharmaceutically acceptable derivative thereof in the treatment of respiratory diseases.

By the expression "PDE4 inhibitor" is meant a selective phosphodiesterase inhibitor, which inhibits preferentially the type 4 phosphodiesterase when compared to other known types of phosphodiesterase, e.g. type 1, 2, 3, 5 etc., whereby the compound has a lower IC₅₀ (more potent) for the type 4 phosphodiesterase, such as where the IC₅₀ for PDE4 inhibition is about factor 10 lower compared to IC₅₀ for inhibition of other known type of phosphodiesterase, e.g. type 1, 2, 3, 5 etc.

Analogously, the expression "PDE3/4 inhibitor" is defined. Methods to determine the activity and selectivity of a phosphodiesterase inhibitor are known to the person skilled in the art. In this connection it may be mentioned, for example, the methods described by Thompson et al. (Adv Cycl Nucl Res 10: 69-92, 1979), Giembycz et al. (Br J Pharmacol 118: 1945-1958, 1996) and the phosphodiesterase scintillation proximity assay of Amersham Pharmacia Biotech.

By the expression "histamine receptor antagonist" are meant H₁-receptor antagonists, particularly the so called H₁-receptor antagonists of the second generation (Mutschler, Arzneimittelwirkungen, 8. Edition, 2001, pages 456-461).

Roflumilast is shown with the aid of its formula:

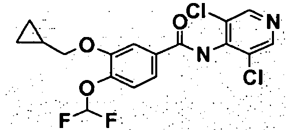

### Roflumilast

In the above formula there is given neither any stereochemical information nor are hydrogen atoms indicated [-O is accordingly -OH, >N is >NH or -N is NH₂]. Methyl groups, e.g. on the oxygen atoms, are indicated by lines].

Substances having good oral availability are preferred here.

In the context of the present invention, unless otherwise stated, a pharmaceutically acceptable derivative of an active ingredient means a pharmaceutically acceptable salt or solvate (e. g. hydrate), a pharmaceutically acceptable solvate of such salt, a pharmaceutically acceptable N-oxide or a pharmaceutically acceptable salt or solvate of the latter.

Suitable pharmacologically tolerable salts here are on the one hand in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid, the acids being employed in salt preparation - depending on whether it is a mono- or polybasic acid and depending on which salt is desired - in an equimolar quantitative ratio or one differing therefrom. Furthermore, the active compounds mentioned can also be present as pure enantiomers or as enantiomer mixtures in any mixing ratio.

On the other hand, salts with bases are also suitable. Examples of salts with bases which may be mentioned are alkali metal (lithium, sodium, potassium) or calcium, aluminium, magnesium, titanium, ammonium, meglumine or guanidinium salts, where here too the bases are employed in salt preparation in an equimolar quantitative ratio or one differing therefrom.

Certain of the active ingredients used in the present invention are capable of existing in stereoisomeric forms. The invention encompasses all stereoisomers of the active ingredients and mixtures thereof including racemates. Tautomers and mixtures thereof of the active ingredients are also part of the present invention.

In accordance with the present invention, there is provided in a first aspect a pharmaceutical composition comprising, in admixture, 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and [2-[4-[(R)-p-chloro-alpha-phenylbenzyl)-1-piperazinyl]ethoxy]-acetic acid [INN: LEVOCETIRIZINE] or a pharmaceutically acceptable derivative thereof.

In a second aspect the invention provides a pharmaceutical product comprising, in combination, a preparation of 3-Cyclopropylmethoxy-4-difluoromethoxy-N-(3,5-dichloropyrid-4-yl)-benzamide [INN: ROFLUMILAST], and a preparation [2-[4-[(R)-p-chloro-alpha-phenylbenzylyl-piperazinyl]ethoxy]-acetic acid [INN: LEVOCETIRIZINE] or a pharmaceutically acceptable derivative thereof for simultaneous, sequential or separate use in therapy.

In a third aspect the invention provides a kit comprising a preparation of 3-Cyclopropylmethoxy-4-difluoromethoxy-N-(3,5-dichloropyrid-4-yl)-benzamide [INN: ROFLUMILAST], and a preparation of [2-[4-[(R)-p-chloro-alpha-phenylbenzyl)-1-piperazinyl]ethoxy]-acetic acid [INN: LEVOCETIRIZINE] or a pharmaceutically acceptable derivative thereof, and instructions for the simultaneous, sequential or separate administration of the preparations to a patient in need thereof.

It has been found that the administration of active ingredients according to the invention is advantageous because it results - in comparison to the administration of a single active ingredient from the PDE4 and PDE3/4 inhibitors or the histamine receptor antagonists class - in a reduced early allergic response and/or in a reduced late inflammatory airway response.

The pharmaceutical composition of the present invention may be prepared by mixing the first active ingredient with the second active ingredient. Therefore, in the fourth aspect of the present invention, there is provided a process for the preparation of a pharmaceutical composition which comprises mixing the first active ingredient 3-Cyclopropylmethoxy-4-difluoromethoxy-N-(3,5-dichloropyrid-4-yl)-benzamide [INN: ROFLUMILAST] or a pharmaceutically acceptable derivative thereof with the second active ingredient 2-[4-[(R)-p-chloro-alpha-phenylbenzyl)-1-piperazinyl]ethoxy]-acetic acid [INN: LEVOCETIRiZINE] or a pharmaceutically acceptable derivative thereof.

The first and second active ingredient may alternatively (other than in admixture as described above) be administered simultaneously, sequentially or separately to treat respiratory diseases. By sequential is meant that the first and second active ingredient are administered one immediately after the other. Separately means a time difference of administration of up to 24 h, preferably up to 12 h.

The present invention further provides the use of a pharmaceutical composition, or pharmaceutical product according to the invention in the manufacture of a medicament for the prophylaxis and/or treatment of a respiratory disease.

Respiratory diseases which may be mentioned are in particular allergen- and inflammation- induced bronchial disorders (bronchitis, obstructive bronchitis, spastic bronchitis, allergic bronchitis, allergic asthma, bronchial asthma, COPD, allergic, seasonal and perennial rhinitis), which can be treated by the combination according to the invention also in the sense of a long-term therapy (if desired with appropriate adjustment of the dosage of the individual components to the needs at the time, for example needs subject to seasonally related variations).

The active ingredients may, and indeed will, as part of the pharmaceutical composition, the Pharmaceutical product or preparation, be used in admixture with one or more pharmaceutically acceptable auxiliaries and/or excipients.

Within the meaning of the present invention, "use" is preferably understood as meaning the oral administration of both active ingredients. The active ingredients also can be administered as a nasal spray, an aerosol, or as an inhaled powder. Further methods of administration, which may be mentioned are the parenteral (e. g. intravenous) and the transdermal administration of the active ingredients.

The invention encompasses on the one hand co-administering both drugs in one delivery form such as a fixed oral combination (putting both active ingredients in one tablet), as an inhaler (putting both active ingredients in the same inhaler) or as a free oral combination (putting both active ingredients in two separate tablets). On the other hand it encompasses also the administration of the drugs in two different delivery forms such as putting the PDE4 inhibitor into tablets and package them with an inhaler that contains the histamine receptor antagonist, or vice versa.

The person skilled in the art is familiar on the basis of his/her expert knowledge with which excipients or auxiliaries are suitable for the desired pharmaceutical composition, product or preparation. In addition to solvents, gel-forming agents, tablet excipients and other active compound carriers, it is possible to use, for example, antioxidants, dispersants, emulsifiers, antifoams, flavor corrigents, preservatives, solubilizers, colorants or permeation promoters and complexing agents (e.g. cyclodextrins).

The pharmaceutical compositions or preparations according to the invention for oral administration are preferably in the form of tablets, coated tablets, capsules, emulsions, suspensions or solutions, the active ingredient content advantageously being between 0.1 and 95% and by appropriate choice of the excipients and the auxiliaries, it being possible to achieve a pharmaceutical administration form precisely tailored to the active ingredient(s) and/or to the desired onset of action (e.g. a sustained release form or an enteric form).

The active ingredients according to the invention are administered by inhalation preferably in the form of an aerosol; the aerosol particles of solid, liquid or mixed composition preferably having a diameter of 0.5 to 10 µm, advantageously of 2 to 6 µm.

Aerosol generation can be carried out, for example, by pressure-driven jet atomizers or ultrasonic atomizers, but advantageously by propellant-driven metered aerosols or propellant-free administration of micronized active compounds from inhalation capsules.

Depending on the inhaler system used, in addition to the active compounds the administration forms additionally contain the required excipients, such as, for example, propellants (e.g. Frigen in the case of metered aerosols), surface-active substances, emulsifiers, stabilizers, preservatives, flavorings, fillers (e.g. lactose in the case of powder inhalers) or, if appropriate, further active compounds.

For the purposes of inhalation, a large number of apparatuses are available with which aerosols of optimum particle size can be generated and administered, using an inhalation technique which is as right as possible for the patient. In addition to the use of adaptors (spacers, expanders) and pear-shaped containers (e.g. Nebulator®, Volumatic®), and automatic devices emitting a puffer spray (Autohaler®), for metered aerosols, in particular in the case of powder inhalers, a number of technical solutions are available (e.g. Diskhaler®, Rotadisk®, Turbohaler® or the inhaler described in European Patent Application EP 0 505 321), using which an optimal administration of active compound can be achieved.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical transdermal formulations comprise a conventional aqueous or non aqueous vehicle, for example a cream, ointment, lotion or paste, or are in the form of a medicated plaster, patch or membrane.

For the above-mentioned therapeutic uses the dosages administered will, of course, vary with the first and second active ingredients employed, the mode of administration, the treatment desired and the disorder indicated.

However, in general, satisfactory results will be obtained when the total daily dosage of first active ingredient(s), the PDE4 respectively the PDE3/4 inhibitors, when taken oral is in the range from 1 - 2000 µg/kg of body weight. In the case of the PDE4 inhibitor ROFLUMILAST, the daily dosage is in a range from 1 - 20 µg/kg of body weight.

The total daily dosage of the second active ingredient(s), the histamine receptor antagonists also can vary within a wide range (0.1- 1500 µg/kg of body weight).

## Claims

1. A pharmaceutical composition comprising, in admixture, 3-Cyclopropylmethoxy-4-difluoromethoxy-N-(3,5-dichtoropyrid-4-yl)-benzamide [INN: ROFLUMILAST], a pharmaceutically acceptable salt, solvate or N-oxide thereof, or solvate of an salt or N-oxide thereof, and [2-[4-[(R)-p-chloro-alpha-phenylbenzyl)-1-piperazinyl]ethoxy]-acetic acid [INN: LEVOCETIRIZINE] or a pharmaceutically acceptable derivative thereof.

2. Use of a pharmaceutical composition according to claim 1 in the manufacture of a medicament for the treatment of respiratory diseases.

3. A process for the preparation of a pharmaceutical composition as defined in claim 1 which comprises mixing 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and [2-[4-[(R)-p-chloro-alpha-phenylbenzyl)-1-piperazinyl]ethoxy]-acetic acid [INN: LEVOCETIRIZINE] or a pharmaceutically acceptable derivative thereof.

4. A pharmaceutical product comprising, in combination, a preparation of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof, and a preparation of [2-[4-[(R)-p-chloro-alpha-phenylbenzyl)-1-piperazinyl]ethoxy]-acetic acid [INN: LEVOCETIRIZINE] or a pharmaceutically acceptable derivative thereof, for simultaneous, sequential or separate use in therapy.

5. Use of a pharmaceutical product according to claim 4 in the manufacture of a medicament for the treatment of respiratory diseases.

6. A kit comprising a preparation of 3-Cyclopropylmethoxy-4-difluoromethoxy-N-(3,5-dichloropyrid-4-yl)-benzamide [INN: ROFLUMILAST], a pharmaceutically acceptable salt, solvate or N-oxide thereof, or solvate of an salt or N-oxide thereof" a preparation of [2-[4-[(R)-p-chloro-alpha-phenylbenzyl)-1-piperazinyl]ethoxy]-acetic acid [INN: LEVOCETIRIZINE] or a pharmaceutically acceptable derivative thereof, and instructions for simultaneous, sequential or separate administration to the patient in need thereof.

7. Use according to any of claims 2, and 5, wherein the respiratory disease is selected from broonchitis, spastic bronchitis, allergic bronchitis, allergic asthma, bronchial asthma, COPD, and allergic, seasonal or perennial rhinitis.

## Patentansprüche

1. Pharmazeutische Zusammensetzung, enthaltend, als Mischung, 3-Cyclopropylmethoxy-4-difluormethoxy-N-(3,5-dichlorpyrid-4-yl)benzamid [INN: ROFLUMILAST], ein pharmazeutisch unbedenkliches Salz, Solvat oder N-Oxid davon, oder ein Solvat eines Salzes oder eines N-Oxids davon, und [2-[4-[(R)-p-Chlor-alpha-phenylbenzyl]-1-piperazinyl]ethoxy]essigsäure [INN: LEVOCETIRIZINE] oder ein pharmazeutisch unbedenkliches Derivat davon.

2. Verwendung einer pharmazeutischen Zusammensetzung nach Anspruch 1 bei der Herstellung eines Medikaments zur Behandlung von Atemwegserkrankungen.

3. Verfahren zur Herstellung einer wie in Anspruch 1 definierten pharmazeutischen Zusammensetzung, bei dem man 3-Cyclopropylmethoxy-4-difluormethoxy-N-(3,5-dichlorpyrid-4-yl)benzamid [INN: ROFLUMILAST], ein pharmazeutisch unbedenkliches Salz, Solvat oder N-Oxid davon, oder ein Solvat eines Salzes oder eines N-Oxids davon, und [2-[4-[(R)-p-Chlor-alpha-phenylbenzyl]-1-piperazinyl]ethoxy]essigsäure [INN: LEVOCETIRIZINE] oder ein pharmazeutisch unbedenkliches Derivat davon mischt.

4. Pharmazeutisches Produkt, enthaltend, als Kombination, eine Zubereitung von 3-Cyclopropylmethoxy-4-difluormethoxy-N-(3,5-dichlorpyrid-4-yl)benzamid [INN: ROFLUMILAST], eines pharmazeutisch unbedenklichen Salzes, Solvates oder N-Oxids davon, oder eines Solvates eines Salzes oder N-Oxids davon und eine Zubereitung von [2-[4-[(R)-p-Chlor-alpha-phenylbenzyl]-1-piperazinyl]ethoxy]essigsäure [INN: LEVOCETIRIZINE] oder eines pharmazeutisch unbedenklichen Derivats davon zur gleichzeitigen, aufeinanderfolgenden oder getrennten Verwendung in der Therapie.

5. Verwendung eines pharmazeutischen Produkts nach Anspruch 4 bei der Herstellung eines Medikaments zur Behandlung von Atemwegserkrankungen.

6. Kit, enthaltend eine Zubereitung von 3-Cyclopropylmethoxy-4-difluormethoxy-N-(3,5-dichlorpyrid-4-yl)benzamid [INN: ROFLUMILAST], eines pharmazeutisch unbedenklichen Salzes, Solvates oder N-Oxids davon, oder eines Solvates eines Salzes oder N-Oxids davon, eine Zubereitung von [2-[4-[(R)-p-Chlor-alpha-phenylbenzyl]-1-piperazinyl]ethoxy]essigsäure [INN: LEVOCETIRIZINE] oder eines pharmazeutisch unbedenklichen Derivats davon und Anweisungen für die gleichzeitige, aufeinanderfolgende oder getrennte Verabreichung an den dessen bedürftigen Patienten.

7. Verwendung nach einem der Ansprüche 2 und 5, wobei die Atemwegserkrankung aus Bronchitis, spastischer Bronchitis, allergischer Bronchitis, allergischem Asthma, Asthma bronchiale, chronisch-obstruktiver Atemwegserkrankung (Chronic-Obstructive Pulmonary Disease, COPD) und allergischer, saisonaler oder perennialer Rhinitis ausgewählt ist.

## Revendications

1. Composition pharmaceutique comprenant, en mélange, le 3-cyclopropylméthoxy-4-difluorométhoxy-N-(3,5-dichloropyrid-4-yl)-benzamide [DCI : ROFLUMILAST], un sel, un solvate ou un N-oxyde pharmaceutiquement acceptable de celui-ci, ou un solvate d'un sel ou d'un N-oxyde de celui-ci, et l'acide [2-[4-[(R)-p-chloro-alpha-phénylbenzyl)-1-pipérazinyl]éthoxy]-acétique [DCI : LEVOCETIRIZINE] ou un dérivé pharmaceutiquement acceptable de celui-ci.

2. Utilisation d'une composition pharmaceutique selon la revendication 1, dans la fabrication d'un médicament destiné au traitement de maladies respiratoires.

3. Procédé de préparation d'une composition pharmaceutique telle que définie dans la revendication 1, **caractérisé en ce qu'**il comprend le mélange du 3-cyclopropylméthoxy-4-difluorométhoxy-N-(3,5-dichloropyrid-4-yl)-benzamide [DCI : ROFLUMILAST], d'un sel, d'un solvate ou d'un N-oxyde pharmaceutiquement acceptable de celui-ci, ou d'un solvate d'un sel ou d'un N-oxyde de celui-ci, et de l'acide [2-[4-[(R)-p-chloro-alpha-phénylbenzyl)-1-pipérazinyl]éthoxy]-acétique [DCI :
LEVOCETIRIZINE] ou d'un dérivé pharmaceutiquement acceptable de celui-ci.

4. Produit pharmaceutique comprenant, en combinaison, une préparation de 3-cyclopropylméthoxy-4-difluorométhoxy-N-(3,5-dichloropyrid-4-yl)-benzamide [DCI : ROFLUMILAST], d'un sel, d'un solvate ou d'un N-oxyde pharmaceutiquement acceptable de celui-ci, ou d'un solvate d'un sel ou d'un N-oxyde de celui-ci, et une préparation de l'acide [2-[4-[(R)-p-chloro-alpha-phénylbenzyl)-1-pipérazinyl]éthoxy]-acétique [DCI : LEVOCETIRIZINE] ou d'un dérivé pharmaceutiquement acceptable de celui-ci, destiné à être utilisé simultanément, séquentiellement ou séparément en thérapie.

5. Utilisation d'un produit pharmaceutique selon la revendication 4, dans la fabrication d'un médicament destiné au traitement de maladies respiratoires.

6. Kit comprenant une préparation de 3-cyclopropylméthoxy-4-difluorométhoxy-N-(3,5-dichloropyrid-4-yl)-benzamide [DCI : ROFLUMILAST], d'un sel, d'un solvate ou d'un N-oxyde pharmaceutiquement acceptable de celui-ci, ou d'un solvate d'un sel ou d'un N-oxyde de celui-ci, et une préparation de l'acide [2-[4-[(R)-p-chloro-alpha-phénylbenzyl)-1-pipérazinyl]éthoxy]-acétique [DCI : LEVOCETIRIZINE] ou d'un dérivé pharmaceutiquement acceptable de celui-ci, et les instructions pour une administration simultanée, séquentielle ou séparée aux patients en ayant besoin.

7. Utilisation selon l'une ou l'autre des revendications 2 et 5, **caractérisée en ce que** la maladie respiratoire est choisie parmi la bronchite, la bronchite spasmodique, la bronchite allergique, l'asthme allergique, l'asthme bronchique, la BPCO et la rhinite allergique, saisonnière ou perannuelle.
